# EUROPEAN PATENT APPLICATION

(11) **EP 4 118 967 A1**
(43) Date of publication of application: **18.01.2023**
(21) Application number: 21768012.3
(22) Date of filing: 10.02.2021
(51) Int. Cl.: A01N 1/02

(54) **PERFUSION DEVICE**

(30) Priority: 10.03.2020 JP 2020040687
(71) Applicant: SCREEN Holdings Co., Ltd., Kyoto 602-8585 (JP)
(72) Inventor: KASAMATSU Hiroo, Kyoto-shi, Kyoto 602-8585 (JP); YOSHIMOTO Syuhei, Kyoto-shi, Kyoto 602-8585 (JP)
(74) Representative: Kilian Kilian & Partner
(86) International application number: PCT/JP2021/004892
(87) International publication number: WO 2021/181985

(57) **Abstract**

A perfusion apparatus (1) includes a reservoir (20) and a perfusion-liquid feeding path (30) that feeds a perfusion liquid from the reservoir (20) to an organ (9). The perfusion-liquid feeding path (30) includes a feeding pipe (31) and a deaeration unit (32) that is interposed in the feeding pipe (31) and includes an air chamber (321) and a deaeration pump (323). The deaeration pump (323) is a roller tube pump in which a plurality of rollers squeeze a flexible gas pipe while rotating, to cause a flow of gas in the gas pipe, and discharges gas from a gas layer in an upper portion of the air chamber (321) to outside. This makes it possible to discharge gas in the air chamber (321) while keeping the perfusion-liquid feeding path (30) hermetic. Therefore, it is possible to remove bubbles in the perfusion liquid fed to the organ (9) without limiting the height of the organ (9) and without stopping feeding the perfusion liquid to the organ (9).

## Description

### Technical Field

The present invention relates to a perfusion apparatus that performs a perfusion process on an extracted organ.

### Background Art

In a transplantation operation such as liver transplantation, an organ is temporarily preserved for a duration from extraction of the organ from a donor to transplantation of the organ into a recipient. There have been developed various preservation methods and perfusion methods to preserve an extracted organ in a transplantable manner. One known method to preserve an extracted organ is, for example, a simple cooling method in which blood in the organ is replaced with a low-temperature organ preservation liquid to suppress metabolism of cells, and then the organ is immersed in the low-temperature preservation liquid. Further, a perfusion preservation method is known in which an organ is perfused with a perfusion liquid by way of a blood vessel network therein for the purpose of removing waste products in the organ being preserved.

A conventional apparatus for preserving an organ outside a body is described in Patent Literature 1, for example.

### Citation List

### Patent Literature

Patent Literature 1: Japanese Patent Application Laid-Open No. H3-151303.

### Summary

### Technical Problem

Patent Literature 1 discloses an apparatus in which a perfusion liquid sucked from a reservoir using a liquid delivery pump is fed to an organ through a perfusion tube. As disclosed, in performing a perfusion process of an organ, pipes are connected to the organ, and a perfusion liquid is fed into the organ.

At that time, it is desirable to remove bubbles in a feeding path of the perfusion liquid to the organ in order to reduce a risk of mixture of air into the organ. The apparatus of Patent Literature 1 is provided with a bubble trap in a feeding path of the perfusion liquid to the organ.

According to a typical deaeration method using an air chamber (bubble trap), a gas layer provided in an upper portion of the air chamber is periodically exposed to the atmosphere, whereby bubbles included in a perfusion liquid are removed. In such an air chamber, during exposure to the atmosphere, a flow of the perfusion liquid is caused due to a head difference with respect to the organ. This necessitates adjustment of the height of the liquid level in the air chamber and the height of the organ.

The present invention has been devised in view of the above-described circumstances, and it is an object of the present invention to provide a technique that can remove bubbles in a perfusion liquid fed to an organ without limiting the height of the organ in a perfusion apparatus.

### Solution to Problem

To solve the above-described problem, the first invention of the present application is directed to a perfusion apparatus that performs a perfusion process on an organ and includes: a reservoir in which a perfusion liquid is stored; and a perfusion-liquid feeding path that feeds the perfusion liquid stored in the reservoir to the organ, wherein the perfusion-liquid feeding path includes: a feeding pipe that delivers the perfusion liquid in the reservoir to the organ; and a deaeration unit that is interposed in the feeding path and removes bubbles in the perfusion liquid, the deaeration unit includes: an air chamber in which the perfusion liquid is temporarily stored; and a deaeration pump that discharges gas from a gas layer in an upper portion of the air chamber to outside of the air chamber, and the deaeration pump is a roller tube pump in which a plurality of rollers squeeze a flexible gas pipe while rotating, to cause a flow of gas in the gas pipe.

The second invention of the present application is directed to the perfusion apparatus according to the first invention, wherein the number of the rollers included in the deaeration pump is three or more.

The third invention of the present application is directed to the perfusion apparatus according to the first invention or the second invention, which further includes: a level sensor that detects a height of a liquid level of the perfusion liquid in the air chamber; and a control unit that drives the deaeration pump when a detection signal from the level sensor indicates that the liquid level of the perfusion liquid is lower than an L level, and stops driving the deaeration pump when the detection signal from the level sensor indicates that the liquid level of the perfusion liquid is higher than an H level higher than the L level.

The fourth invention of the present application is directed to the perfusion apparatus according to any of the first invention to the third invention, which further includes a perfusion-liquid recovery path that causes the perfusion liquid discharged from the organ to flow back to the reservoir, wherein the perfusion-liquid recovery path includes: a recovery pipe that delivers the perfusion liquid from the organ to the reservoir; a liquid delivery pump interposed in the recovery pipe; and a second deaeration unit that is interposed upstream of the liquid delivery pump in the recovery pipe and removes bubbles in the perfusion liquid, the second deaeration unit includes: a second air chamber in which the perfusion liquid is temporarily stored; and a second deaeration pump that discharges gas from a gas layer in an upper portion of the second air chamber to outside of the second air chamber, and the second deaeration pump is a roller tube pump in which a plurality of rollers squeeze a flexible gas pipe while rotating, to cause a flow of gas in the gas pipe.

### Effects of Invention

According to the first invention to the fourth invention of the present application, gas in the air chamber is exhausted using the roller tube pump, whereby the gas in the air chamber can be appropriately discharged with the perfusion-liquid feeding path kept in a closed system (hermetic). Therefore, it is possible to remove bubbles in the perfusion liquid fed to the organ without limiting the height of the organ in the perfusion apparatus and without stopping feeding the perfusion liquid to the organ.

Especially, according to the second invention of the present application, the hermeticity of the deaeration pump is further increased.

Especially, according to the third invention of the present application, the height of the gas layer formed in the upper portion of the air chamber can be kept within a predetermined range.

Especially, according to the fourth invention of the present application, bubbles can be prevented from being mixed into the liquid delivery pump.

### Brief Description of Drawings

Fig. 1 is a schematic view showing a configuration of a perfusion apparatus according to a first embodiment.
Fig. 2 is a plan view of a deaeration pump according to the first embodiment.
Fig. 3 is a schematic view showing a configuration of a perfusion apparatus according to a second embodiment.
Fig. 4 is a schematic view showing a configuration of a perfusion apparatus according to a third embodiment.
Fig. 5 is a plan view of a deaeration pump according to a modification.

### Description of Embodiments

Hereinafter, embodiments of the present invention will be described with reference to the drawings. The term "organ" in the present application may mean a human organ or a non-human organ. Thus, the terms "donor" and "recipient" may mean humans or non-human animals. Further, non-human animals may be rodents including mouses and rats, ungulates including pigs, goats and sheep, non-human primates including chimpanzees, other non-human mammals, or animals other than mammals.

### <1. First Embodiment>

### <1-1. Configuration of Perfusion Apparatus>

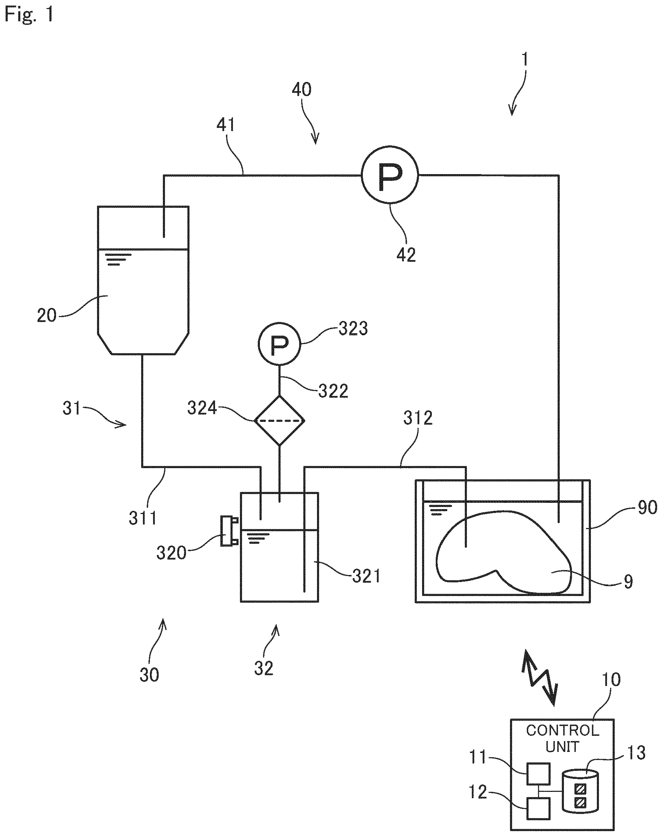

A perfusion apparatus 1 according to a first embodiment of the present invention will be described with reference to Fig. 1. Fig. 1 is a schematic view showing a configuration of the perfusion apparatus 1. The perfusion apparatus 1 is an apparatus for temporarily preserving a liver extracted from a donor, outside a body, until the liver is transplanted to a recipient. The perfusion apparatus 1 feeds a perfusion liquid to the liver, to perform perfusion. Note that, an organ being perfused by the perfusion apparatus 1 is not limited to a liver and may be a kidney, a spleen, or other organs.

In a perfusion process by the perfusion apparatus 1, a liver 9 is contained in a reactor 90. The reactor 90 contains an organ preservation liquid and the liver 9 connected to the perfusion apparatus 1. As the reactor 90, a cup-shaped (bottomed-tube-shaped) container is used, for example.

As shown in Fig. 1, the perfusion apparatus 1 includes a reservoir 20, a perfusion-liquid feeding path 30, a perfusion-liquid recovery path 40, and a control unit 10.

The reservoir 20 is a container in which a perfusion liquid is stored. The reservoir 20 of the present embodiment is a soft bag formed of a flexible container like an intravenous bag. As a perfusion liquid stored in the reservoir 20, an ETK liquid is used, for example. As a perfusion liquid, other kinds of perfusion liquids such as a UW liquid may be used.

Further, there may be provided around the reservoir 20, a temperature regulation mechanism that regulates the temperature of a perfusion liquid stored in the reservoir 20 and a gas exchange mechanism that adds gas such as oxygen to a perfusion liquid stored in the reservoir 20. Meanwhile, such a temperature regulation mechanism and a gas exchange mechanism may be provided in the perfusion-liquid feeding path 30. Note that, in a case in which a gas exchange mechanism is provided in the perfusion-liquid feeding path 30, the gas exchange mechanism is provided upstream of a deaeration unit 32 described later.

The perfusion-liquid feeding path 30 feeds a perfusion liquid from the reservoir 20 to the liver 9 contained in the reactor 90. The perfusion-liquid feeding path 30 includes a feeding pipe 31 and the deaeration unit 32 interposed in the feeding pipe 31.

The feeding pipe 31 is a pipe for feeding a perfusion liquid from the reservoir 20 to the liver 9 contained in the reactor 90. The feeding pipe 31 includes a first feeding pipe 311 and a second feeding pipe 312.

The first feeding pipe 311 is a pipe for feeding a perfusion liquid from the reservoir 20 to the deaeration unit 32. One end of the first feeding pipe 311 is connected to the lower portion of the reservoir 20 to communicate with the reservoir 20. The other end of the first feeding pipe 311 is connected to an air chamber 321 described later in the deaeration unit 32 to communicate therewith.

The second feeding pipe 312 is a pipe for feeding a perfusion liquid from the deaeration unit 32 to the liver 9 contained in the reactor 90. One end of the second feeding pipe 312 is connected to the lower portion of the air chamber 321 to communicate with the air chamber 321. The other end of the second feeding pipe 312 is connected to a blood vessel for inflow of a perfusion liquid in the liver 9. Specifically, the other end of the second feeding pipe 312 is ligated to a portal vein or a hepatic artery of the liver 9, so that it is connected to communicate with the liver 9.

The deaeration unit 32 includes the air chamber 321, a deaeration pipe 322, a deaeration pump 323, and a hydrophobic filter 324.

The air chamber 321 is a container for temporarily storing a perfusion liquid therein and separating the perfusion liquid itself and bubbles included in the perfusion liquid from each other. The air chamber 321 is provided with a level sensor 320 that detects the height of the liquid level of a perfusion liquid in the air chamber 321. As the level sensor 320, an optical level sensor that projects and receives light onto and from the air chamber 321 to recognize the presence of a perfusion liquid, is used, for example. The control unit 10 controls the deaeration pump 323 such that a certain gas layer is formed in the upper portion of the air chamber 321, in accordance with a signal from the level sensor 320.

The deaeration pipe 322 is a pipe for discharging gas from the gas layer in the upper portion of the air chamber 321. One end of the deaeration pipe 322 is connected to the gas layer in the upper portion of the air chamber 321 to communicate therewith. The other end of the deaeration pipe 322 is connected to the deaeration pump 323. Details of the deaeration pump 323 will be given later.

The hydrophobic filter 324 is interposed in the deaeration pipe 322. Because of this, a perfusion liquid or moisture in the air chamber 321 is prevented from erroneously flowing toward the deaeration pump 323.

The reservoir 20 is placed above the air chamber 321 and the liver 9. A perfusion liquid in the reservoir 20 is fed to the liver 9 through the perfusion-liquid feeding path 30 due to a head difference between the air chamber 321 and the liver 9. The perfusion liquid fed to the liver 9 circulates through the liver 9, and then flows out to the reactor 90 from a blood vessel for discharging blood from the liver 9, such as supra-hepatic inferior vena cava (SH-IVC) or infra-hepatic inferior vena cava (IH-IVC).

The perfusion-liquid recovery path 40 recovers a perfusion liquid that is discharged from the liver 9 and stored in the reactor 90, and causes the perfusion liquid to flow back to the reservoir 20. The perfusion-liquid recovery path 40 includes a recovery pipe 41 and a liquid delivery pump 42.

The recovery pipe 41 is a pipe for delivering a perfusion liquid from the reactor 90 to the reservoir 20. One end of the recovery pipe 41 is placed in a perfusion liquid stored in the lower portion of the reactor 90. The other end of the recovery pipe 41 is connected to the reservoir 20 to communicate therewith.

The liquid delivery pump 42 is interposed in the recovery pipe 41. The liquid delivery pump 42 causes a flow of a perfusion liquid from the reactor 90 toward the reservoir 20 in the recovery pipe 41. As the liquid delivery pump 42, a centrifugal pump with an impeller is used, for example. When the liquid delivery pump 42 is driven, a perfusion liquid stored in the reactor 90 is caused to flow back to the reservoir 20.

The perfusion apparatus 1 of the present embodiment has a configuration in which a perfusion liquid discharged from the liver 9 is caused to flow back to the reservoir 20. However, the present invention is not limited to the configuration. A perfusion liquid discharged from the liver 9 may be disposed of or stored in another container, instead of being caused to flow back to the reservoir 20.

Note that, each of the reservoir 20, the perfusion-liquid feeding path 30, and the perfusion-liquid recovery path 40 may be provided with a measurement unit for detecting pH or a specific component, as appropriate. Further, a pressure gauge, a flowmeter, a solenoid valve that controls communication, and the like may be interposed in the feeding pipe 31 and the recovery pipe 41.

The control unit 10 is a unit for controlling the operations of each part in the perfusion apparatus 1. As conceptually shown in Fig. 1, the control unit 10 is formed of a computer including an arithmetic processing unit 11 such as a CPU, a memory 12 such as a RAM, and a storage unit 13 such as a hard disk drive.

### <1-2. Deaeration Unit>

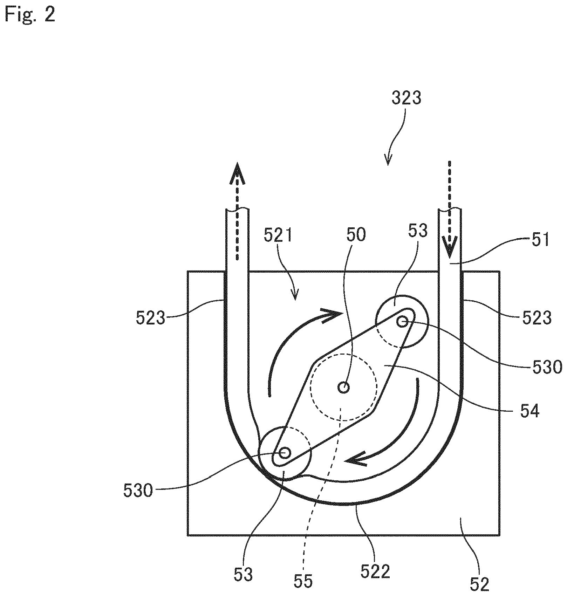

Next, the deaeration unit 32 will be described in detail with reference to Fig. 1 and Fig. 2. First, the deaeration pump 323 included in the deaeration unit 32 will be described with reference to Fig. 2. Fig. 2 is a plan view of the deaeration pump 323 of the present embodiment.

As shown in Fig. 2, the deaeration pump 323 includes a gas pipe 51, a frame 52 fixing the gas pipe 51, a plurality of rollers 53, a roller holder 54, and a motor 55. The deaeration pump 323 is a so-called a roller tube pump in which the rollers 53 squeeze the gas pipe 51 while rotating, to cause a flow of gas in the gas pipe 51.

In the roller tube pump, a flexible pipe is squeezed using a roller and the roller is moved with the flexible pipe being kept squeezed, so that a fluid in the flexible pipe is sucked from the upstream side. Thus, the roller tube pump can keep the upstream-side portion of the roller tube pump in a closed state (hermetic) without a need for a check valve.

The gas pipe 51 is a flexible pipe formed of elastomer. An upstream-side end of the gas pipe 51 is connected to a downstream-side end of the deaeration pipe 322 to communicate therewith. Note that, the gas pipe 51 and the deaeration pipe 322 may be formed integrally with each other.

The frame 52 is a member for fixing the periphery of the gas pipe 51 that is placed while being bent. The frame 52 includes a recessed portion 521 in which the gas pipe 51 is placed. The edge of the recessed portion 521 includes an arc-shaped portion 522 in a shape of an arc having its center at a rotation center 50 and an open portion 523 that extends from both ends of the arc-shaped portion 522 to the ends of the frame 52 and is open.

During use of the deaeration pump 323, the gas pipe 51 is placed along the edge of the recessed portion 521 of the frame 52. Thus, the gas pipe 51 is placed while being bent along the edge of the recessed portion 521. Consequently, the bent periphery of the gas pipe 51 extends along the edge of the recessed portion 521.

The plurality of rollers 53 are mounted in the roller holder 54 that rotates about the rotation center 50. The deaeration pump 323 of the present embodiment includes two rollers 53. The number of rollers 53 included in the deaeration pump 323 may be two, or three or more.

The two rollers 53 are mounted rotatably about roller shafts 530 with respect to the roller holder 54. Thus, the roller shafts 530 revolve about the rotation center 50 as the roller holder 54 rotates, and when the surfaces of the rollers 53 come into contact with the gas pipe 51, the rollers 53 rotate about the roller shafts 530 so as to roll on the surface of the gas pipe 51.

The rollers 53 and the roller holder 54 are placed on the inner-surface side of the gas pipe 51 that is placed while being bent along the recessed portion 521. The roller holder 54 is caused to rotate about the rotation center 50 by the motor 55. As the motor 55, a brushless motor is used, for example. In Fig. 2, the rotation direction of the rollers 53 and the roller holder 54 is indicated by solid-line arrows. Further, a direction in which gas flows in the gas pipe 51 is indicated by broken-line arrows.

The two rollers 53 are placed so as to be 180° rotationally symmetric with respect to the rotation center 50. The arc-shaped portion 522 of the frame 52 has a shape of an arc having its center at the rotation center 50. Further, a difference between a distance between the rotation center 50 and the arc-shaped portion 522 and a distance between the rotation center 50 and the outer end of the roller 53 is smaller than the outer diameter of the gas pipe 51. More specifically, a difference between a distance between the rotation center 50 and the arc-shaped portion 522 and a distance between the rotation center 50 and the outer end of the roller 53 is substantially the same as twice the thickness of a tube of the gas pipe 51. For this reason, the rollers 53 rotate while pressing the gas pipe 51 to squeeze it on the inner side of the arc-shaped portion 522. As a result, gas in the gas pipe 51 is forced out in the rotation direction of the rollers 53 and the roller holder 54, so that a flow of gas is caused in the gas pipe 51.

When the rollers 53 press the gas pipe 51 on the inner side of the arc-shaped portion 522, the gas pipe 51 becomes blocked. Further, the central angle of the arc-shaped portion 522 having its center at the rotation center 50 is equal to 180° or more. For this reason, at least one of the two rollers 53 is placed on the inner side of the arc-shaped portion 520. Consequently, in the deaeration pump 323, at least one portion of the gas pipe 51 is blocked, so that hermeticity is secured.

The air chamber 321 is provided with the level sensor 320 as described above. In the present embodiment, the level sensor 320 recognizes the presence of a perfusion liquid at the heights of two positions, an H level and an L level, in the air chamber 321. The H level indicates a position higher than the L level. In a case in which gas is mixed into a perfusion liquid fed from the first feeding pipe 31, the gas is trapped in the air chamber 321, so that the height of the liquid level of the perfusion liquid in the air chamber 321 gradually deceases.

The control unit 10 drives the deaeration pump 323 when the control unit 10 determines that a detection signal from the level sensor 320 indicates that the liquid level of a perfusion liquid is lower than the L level. Meanwhile, the control unit 10 stops driving the deaeration pump 323 when the control unit 10 determines that a detection signal from the level sensor 320 indicates that the liquid level of a perfusion liquid is higher than the H level. Thus, the height of the liquid level of a perfusion liquid in the air chamber 321 can be kept between the H level and the L level. As a result, the height of the gas layer formed in the upper portion of the air chamber 321 can be kept within a predetermined range.

In a typical deaeration unit that discharges gas stored in an air chamber by exposure to the atmosphere, a flow of a perfusion liquid is caused due to a head difference with respect to an organ during exposure to the atmosphere, which necessitates adjustment of the heights of the liquid level of the perfusion liquid in the air chamber and the organ. Further, in some cases, a perfusion liquid stops flowing through a perfusion-liquid feeding path during exposure to the atmosphere.

The perfusion apparatus 1, in which gas in the air chamber 321 is exhausted using the deaeration pump 323 that is a roller tube pump, can appropriately discharge the gas in the air chamber 321 while keeping the perfusion-liquid feeding path 30 in a closed state (hermetic). Therefore, it is possible to remove bubbles in a perfusion liquid fed to an organ (the liver 9) without limiting the height of the organ (the liver 9) in the perfusion apparatus 1 and without stopping feeding the perfusion liquid to the organ (the liver 9).

### <2. Second Embodiment>

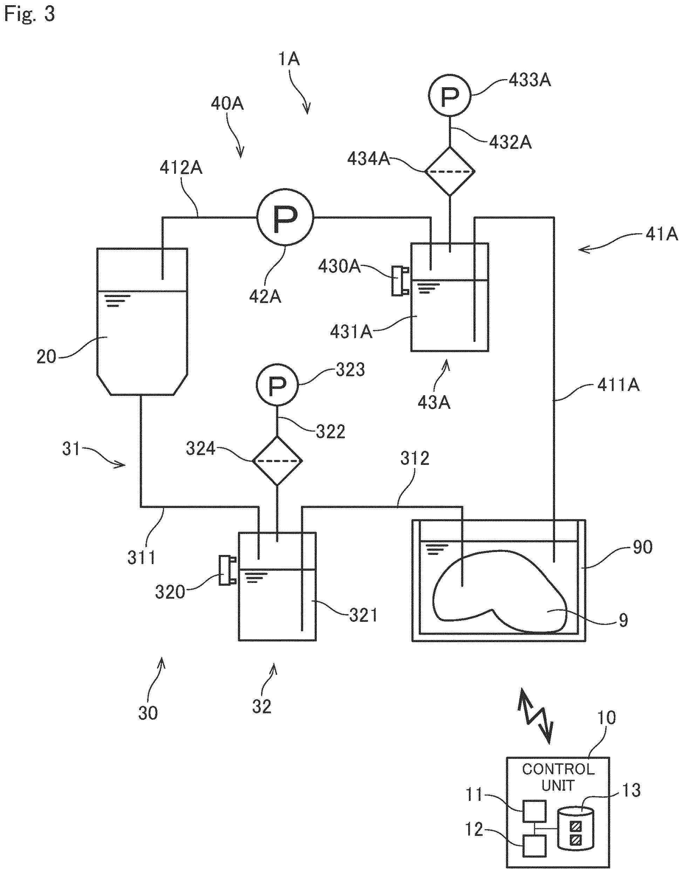

Next, a perfusion apparatus 1A according to a second embodiment of the present invention will be described with reference to Fig. 3. Fig. 3 is a schematic view showing a configuration of the perfusion apparatus 1A. Among components in Fig. 3, components denoted by the same reference signs as those in Fig. 1 are components similar to those in the first embodiment.

A perfusion-liquid recovery path 40A of the perfusion apparatus 1A has a configuration in which a second deaeration unit 43A is interposed in the perfusion-liquid recovery path 40 of the perfusion apparatus 1 according to the first embodiment. Specifically, the perfusion-liquid recovery path 40A includes a recovery pipe 41A, a liquid delivery pump 42A, and the second deaeration unit 43A.

The recovery pipe 41A includes a first recovery pipe 411A that delivers a perfusion liquid discharged from the liver 9 to the second deaeration unit 43A, and a second recovery pipe 412A that delivers the perfusion liquid from the second deaeration unit 43A to the reservoir 20. The liquid delivery pump 42A is interposed in the second recovery pipe 412A. That is, the second deaeration unit 43A is placed upstream of the liquid delivery pump 42A.

The second deaeration unit 43Ahas a configuration similar to that of the deaeration unit 32. The second deaeration unit 43A includes a second air chamber 431A, a second deaeration pipe 432A, a second deaeration pump 433A, and a hydrophobic filter 434A.

The second deaeration pump 433A, like the deaeration pump 323, is a so-called roller tube pump in which a plurality of rollers squeeze a flexible gas pipe while rotating, to cause a flow of gas in the gas pipe.

In a case in which red blood cells are added to a perfusion liquid, it is preferable to use an impeller centrifugal pump in which hemolysis of the red blood cells is unlikely to occur, as the liquid delivery pump 42A. In such a centrifugal pump, mixture of bubbles into the pump hinders rotation of the pump in some cases. In view of this, the second deaeration unit 43A is placed upstream of the liquid delivery pump 42A, whereby bubbles can be prevented from being mixed into the liquid delivery pump 42A even in a case in which bubbles are mixed into a perfusion liquid in the reactor 90.

### <3. Third Embodiment>

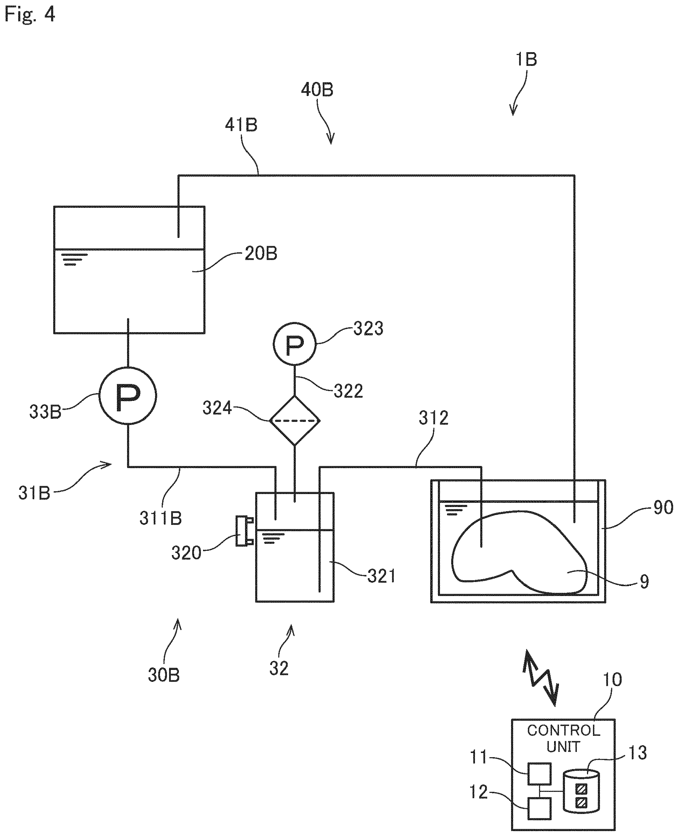

Next, a perfusion apparatus 1B according to a third embodiment of the present invention will be described with reference to Fig. 4. Fig. 4 is a schematic view showing a configuration of the perfusion apparatus 1B. Among components in Fig. 4, components denoted by the same reference signs as those in Fig. 1 are components similar to those in the first embodiment.

In the perfusion apparatus 1B, a reservoir 20B is not a soft bag, but a hard and hermetic container. Further, in the perfusion apparatus 1B, a liquid delivery pump 33B is provided in not a perfusion-liquid recovery path 40B, but a perfusion-liquid feeding path 30B.

Specifically, in the perfusion-liquid feeding path 30B, the liquid delivery pump 33B is interposed in a first feeding pipe 311B that feeds a perfusion liquid from the reservoir 20B to the air chamber 321. Meanwhile, no liquid delivery pump is interposed in a recovery pipe 41B of the perfusion-liquid recovery path 40B.

In the perfusion apparatus 1B described above, when the liquid delivery pump 33B is driven, a perfusion liquid is fed from the reservoir 20B to the liver 9 via the deaeration unit 32. This causes reduction of a pressure of the inside of the reservoir 20B, to reduce a pressure of the inside of the recovery pipe 41B. As a result, the perfusion liquid that is discharged from the liver 9 and is stored in the reactor 90 is sucked into the recovery pipe 41B and then flows back to the reservoir 20B.

As described above, the position of a pump used for liquid delivery may be appropriately changed depending on the kind of the reservoir 20B.

### <4. Modification>

Hereinabove, the embodiments of the present invention have been described. However, the present invention is not limited to the above-described embodiments.

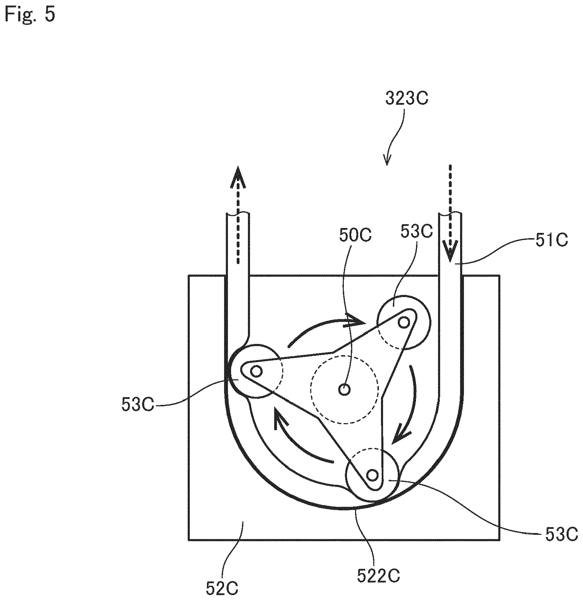

Fig. 5 is a plan view of a deaeration pump 323C according to a modification. The deaeration pump 323C is a pump used as a deaeration pump provided in a perfusion-liquid feeding path. The deaeration pump 323C, like the above-described deaeration pump 323 of the first embodiment, is a so-called roller tube pump in which a plurality of rollers 53C squeeze a gas pipe 51C while rotating, to cause a flow of gas in the gas pipe 51C.

The deaeration pump 323 of the first embodiment includes two rollers 53, but the deaeration pump 323C includes three rollers 53C. The three rollers 53C are placed so as to be 120° rotationally symmetric (threefold symmetric) with respect to a rotation center 50C. Further, an arc-shaped portion 522C of a frame 52C of the deaeration pump 323C, like that of the frame 52 of the first embodiment, has a central angle of 180° having its center at the rotation center 50C.

In a case in which the central angle of the arc-shaped portion 522C is unchanged, as the number of rollers 53C increases, the plurality of rollers 53C are placed more inwardly in the arc-shaped portion 522C, so that a period during which the gas pipe 51C is pressed is lengthened. This can prevent the hermeticity of the deaeration pump 323C as a whole from being reduced also in the unlikely event that the pressing force of the rollers 53C against some portions in the gas pipe 51C is weakened or the rollers 53C include a roller having a weak pressing force against the gas pipe 51C, due to a manufacturing error or a setting error.

Further, though the perfusion-liquid recovery path recovers a perfusion liquid discharged from a liver into the reactor in the above-described embodiments, the present invention is not limited thereto. For example, there may be formed a configuration in which an upstream-side end of a recovery pipe is connected to supra-hepatic inferior vena cava (SH-IVC) or intra-hepatic inferior vena cava (IH-IVC) of a liver, and the perfusion-liquid recovery path 40 recovers a perfusion liquid from the blood vessel connected thereto.

Further, though the perfusion apparatus includes a single perfusion-liquid feeding path and a single perfusion-liquid recovery path in the above-described embodiments, the present invention is not limited thereto. For example, the perfusion apparatus may include two perfusion-liquid feeding paths that feed perfusion liquids to a portal vein and a hepatic artery of a liver, respectively. Moreover, the perfusion apparatus may include two perfusion-liquid recovery paths that recover perfusion liquids from supra-hepatic inferior vena cava (SH-IVC) and intra-hepatic inferior vena cava (IH-IVC) of a liver, respectively.

Furthermore, the respective elements described in the above-described embodiments and modification may be appropriately combined with each other unless contradiction occurs.

### Reference Signs List

- 1, 1A, 1B: Perfusion apparatus
- 9: Liver
- 20, 20B: Reservoir
- 30, 30B: Perfusion-liquid feeding path
- 31: Feeding pipe
- 32: Deaeration pump
- 32: Deaeration unit
- 40, 40A, 40B: Perfusion-liquid recovery path
- 41, 41A, 41B: Recovery pipe
- 42, 42A, 33B: Liquid delivery pump
- 43A: Second deaeration unit
- 51, 51C: Gas pipe
- 53, 53C: Roller
- 321: Air chamber
- 323, 323C: Deaeration pump
- 431A: Second air chamber
- 433A: Second deaeration pump

## Claims

1. A perfusion apparatus that performs a perfusion process on an organ, comprising:
a reservoir in which a perfusion liquid is stored; and
a perfusion-liquid feeding path that feeds the perfusion liquid stored in the reservoir to the organ, wherein
the perfusion-liquid feeding path includes:
a feeding pipe that delivers the perfusion liquid in the reservoir to the organ; and
a deaeration unit that is interposed in the feeding pipe and removes bubbles in the perfusion liquid,
the deaeration unit includes:
an air chamber in which the perfusion liquid is temporarily stored; and
a deaeration pump that discharges gas from a gas layer in an upper portion of the air chamber to outside of the air chamber, and
the deaeration pump is a roller tube pump in which a plurality of rollers squeeze a flexible gas pipe while rotating, to cause a flow of gas in the gas pipe.

2. The perfusion apparatus according to claim 1, wherein the number of the rollers included in the deaeration pump is three or more.

3. The perfusion apparatus according to claim 1 or 2, further comprising:
a level sensor that detects a height of a liquid level of the perfusion liquid in the air chamber; and
a control unit that drives the deaeration pump when a detection signal from the level sensor indicates that the liquid level of the perfusion liquid is lower than an L level, and stops driving the deaeration pump when the detection signal from the level sensor indicates that the liquid level of the perfusion liquid is higher than an H level higher than the L level.

4. The perfusion apparatus according to any of claims 1 to 3, further comprising
a perfusion-liquid recovery path that causes the perfusion liquid discharged from the organ to flow back to the reservoir, wherein
the perfusion-liquid recovery path includes:
a recovery pipe that delivers the perfusion liquid from the organ to the reservoir;
a liquid delivery pump interposed in the recovery pipe; and
a second deaeration unit that is interposed upstream of the liquid delivery pump in the recovery pipe and removes bubbles in the perfusion liquid,
the second deaeration unit includes:
a second air chamber in which the perfusion liquid is temporarily stored; and
a second deaeration pump that discharges gas from a gas layer in an upper portion of the second air chamber to outside of the second air chamber, and
the second deaeration pump is a roller tube pump in which a plurality of rollers squeeze a flexible gas pipe while rotating, to cause a flow of gas in the gas pipe.
